# EUROPEAN PATENT APPLICATION

(11) **EP 2 238 957 A1**
(43) Date of publication of application: **13.10.2010**
(21) Application number: 09005266.3
(22) Date of filing: 10.04.2009
(51) Int. Cl.: A61F 13/535, A61F 13/539, A61F 13/534

(54) **Absorbent core**

(71) Applicant: The Procter and Gamble Company, Cincinnati, Ohio 45202 (US)
(72) Inventor: Carlucci, Giovanni, 66100 Chieti (IT); Tamburro, Maurizio, 66020 Sambuceto (Chieti) (IT)
(74) Representative: Veronese, Pancrazio

(57) **Abstract**

Absorbent core for disposable absorbent articles, for example for the absorption of menses or blood, comprising porous absorbent gelling material.

## Description

### FIELD OF THE INVENTION

The present invention relates to an absorbent core for absorbent articles, for example sanitary napkins and the like.

### BACKGROUND OF THE INVENTION

Absorbent articles for absorption of body fluids such as menses or blood or vaginal discharges are well known in the art, and comprise for example feminine hygiene articles such as sanitary napkins, panty liners, tampons, interlabial devices, as well as wound dressings, and the like. When considering for example sanitary napkins, these articles typically comprise a liquid-pervious topsheet as wearer-facing layer, a backsheet as garment-facing layer and an absorbent core between topsheet and backsheet. The body fluids are acquired through the topsheet and subsequently stored in the absorbent core. The backsheet typically prevents the absorbed fluids from wetting the wearer's garment.

An absorbent core can typically comprise one or more fibrous absorbent materials, which in turn can comprise natural fibres, such as for example cellulose fibres, typically wood pulp fibres, synthetic fibres, or combinations thereof.

Absorbent articles can further comprise, typically in the absorbent core, superabsorbent materials, such as absorbent gelling materials (AGM), usually in finely dispersed form, e.g. typically in particulate form, in order to improve their absorption and retention characteristics. Superabsorbent materials for use in absorbent articles typically comprise water-insoluble, water-swellable, hydrogel-forming crosslinked absorbent polymers which are capable of absorbing large quantities of liquids and of retaining such absorbed liquids under moderate pressure. Absorbent gelling materials can be incorporated in absorbent articles, typically in the core structure, in different ways; for example, absorbent gelling materials in particulate form can be dispersed among the fibres of fibrous layers comprised in the core, or rather localized in a more concentrated arrangement between fibrous layers.

Absorbent cores for absorbent articles having a thin structure can further provide an improved immobilization of absorbent gelling materials, particularly when the article is fully or partially loaded with liquid, and an increased wearing comfort. Such thinner structures provide absorbent articles combining better comfort, discreetness and adaptability, such as for example, thin absorbent structures where the absorbent gelling material is located and somehow kept in selected, e.g. patterned regions of the structure itself.

EP 1447067, assigned to the Procter & Gamble Company, describes an absorbent article, typically a disposable absorbent article, such as a diaper, having an absorbent core which imparts increased wearing comfort to the article and makes it thin and dry. The absorbent core comprises a substrate layer, the substrate layer comprising a first surface and a second surface, the absorbent core further comprising a discontinuous layer of absorbent material, the absorbent material comprising an absorbent polymer material, the absorbent material optionally comprising an absorbent fibrous material which does not represent more than 20 weight percent of the total weight of the absorbent polymer material. The discontinuous layer of absorbent material comprises a first surface and a second surface, the absorbent core further comprising a layer of thermoplastic material, the layer of thermoplastic material comprising a first surface and a second surface and wherein the second surface of the discontinuous layer of absorbent material is in at least partial contact with the first surface of the substrate layer and wherein portions of the second surface of the layer of thermoplastic material are in direct contact with the first surface of the substrate layer and portions of the second surface of the layer of thermoplastic material are in direct contact with the first surface of the discontinuous layer of absorbent material.

While absorbent articles according to EP 1447067 and comprising thin absorbent corcs with relatively high amounts of absorbent gelling materials and rather low content of fibrous materials commonly have good absorption and retention characteristics to body fluids, there still remains room for improvement of absorption and retention, and particularly fluid acquisition capability.

It is believed that the presence of the layer of thermoplastic material, typically for example a hot melt adhesive which can be in fibres, in direct contact with the first surface of the layer of absorbent material, in turn typically for example comprising particles of an absorbent polymer material, while providing a stable absorbent structure and an effective containment of the absorbent polymer material onto the substrate layer, also in wet state upon absorption of fluid, can however have some effect on the behaviour of the absorbent polymer material towards fluids. For example, the layer of thermoplastic material can cover at least partially the outer surface of the absorbent polymer material, typically particulate absorbent polymer material, and hence might have some influence e.g. on its fluid acquisition capacity.

The present invention provides significant improvements in the above area by the incorporation of porous absorbent polymer particles in an absorbent core structure for an absorbent article, which comprises the absorbent gelling material in a layer stably provided onto a substrate layer.

### SUMMARY OF THE INVENTION

The present invention addresses the above needs by providing an absorbent core for an absorbent article, which comprises a substrate layer, comprising a first surface and a second surface; the absorbent core further comprises a layer of absorbent polymer material, comprising a first surface and a second surface; the absorbent core also comprises a layer of adhesive, comprising a first surface and a second surface. The layer of absorbent polymer material is comprised between the layer of hot melt adhesive material and the substrate layer. The second surface of the layer of absorbent polymer material is in contact with the first surface of the substrate layer, and the first surface of the layer of absorbent polymer material is in contact with the second surface of the layer of hot melt adhesive. The layer of absorbent polymer material further comprises porous absorbent polymer particles.

### BRIEF DESCRIPTION OF THE DRAWINGS

Figure 1 is a plan view of a sanitary napkin showing an absorbent core according to an embodiment of the present invention, with portions of some constituent elements cut out in order to show underlying elements.
Figure 2 is a schematic cross section of the sanitary napkin of Figure 1 taken in the transverse axis A-A'.
Figure 3 shows a schematic cross section of an absorbent core according to one embodiment of the present invention.
Figure 4 shows a schematic cross section of an absorbent core according to another embodiment of the present invention.
Figure 5 shows a perspective view of an exemplary absorbent core according to the present invention

### DETAILED DESCRIPTION OF THE INVENTION

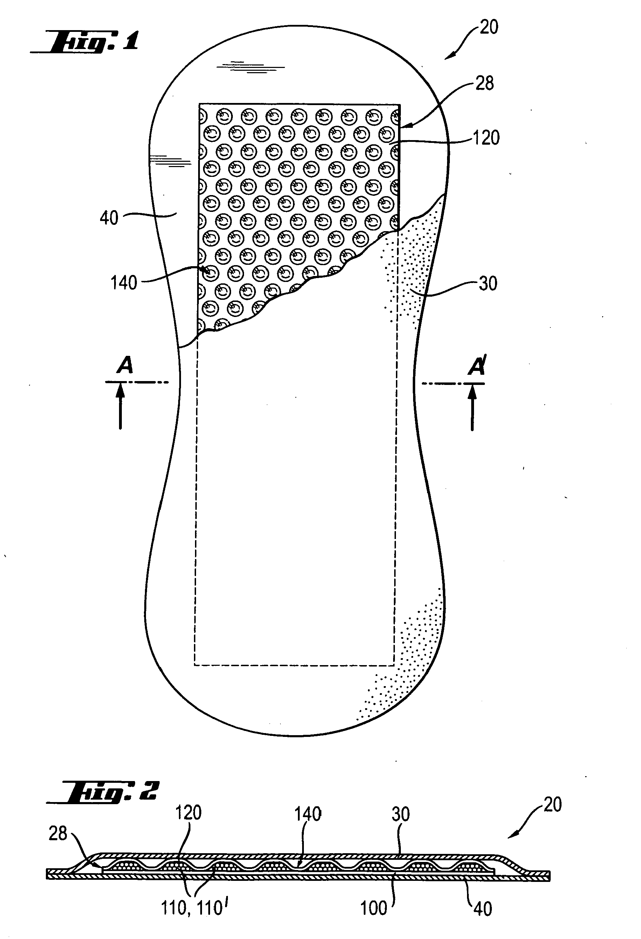

The present invention relates to an absorbent core for absorbent articles such as sanitary napkins, panty liners, tampons, interlabial devices, wound dressings, diapers, adult incontinence articles, and the like, which are intended for the absorption of body fluids, such as menses or blood or vaginal discharges or urine. Exemplary absorbent articles in the context of the present invention are disposable absorbent articles. The term "disposable" is used herein to describe articles, which are not intended to be laundered or otherwise restored or reused as an article (i.e. they are intended to be discarded after a single use and possibly to be recycled, composted or otherwise disposed of in an environmentally compatible manner). The absorbent article comprising an absorbent core according to the present invention can be for example a sanitary napkin or a panty liner. The absorbent core of the present invention will be herein described in the context of a typical absorbent article, such as, for example, a sanitary napkin 20 as illustrated in Figure 1. Typically, such articles as shown in Figure 1 can comprise the elements of a liquid pervious topsheet 30, a backsheet 40 and an absorbent core 28 intermediate said topsheet 30 and said backsheet 40.

In the following description of the invention, the surface of the article, or of each element thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

### Topsheet

According to the present invention, the absorbent article can comprise a liquid pervious topsheet. The topsheet suitable for use herein can comprise wovens, non-wovens, and/or three-dimensional webs of a liquid impermeable polymeric film comprising liquid permeable apertures. In Figure 1 the topsheet is indicated with reference numeral 30. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

### Absorbent Core

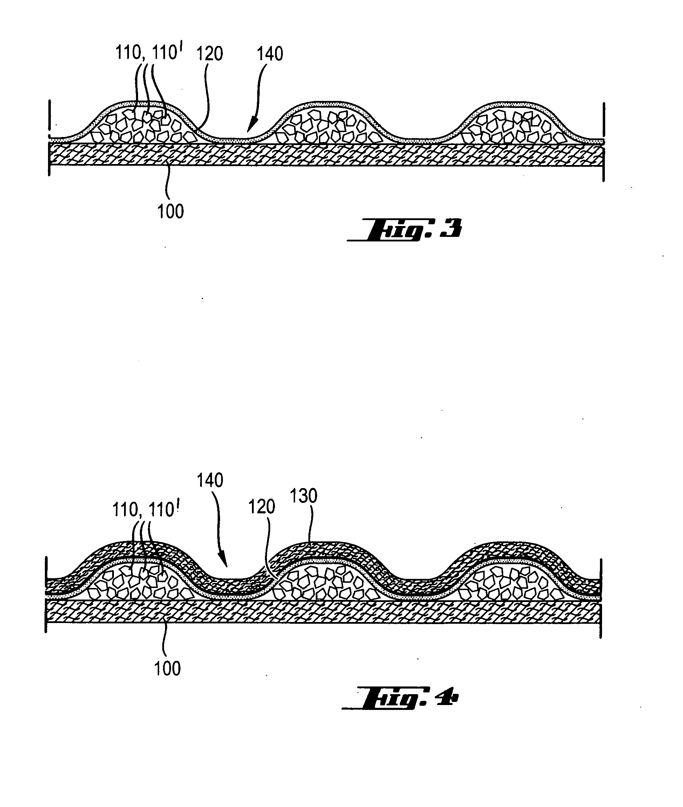

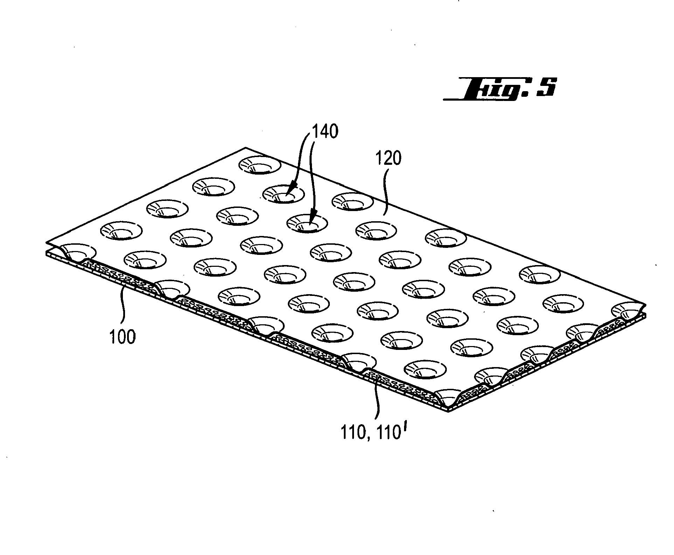

According to an embodiment of the present invention, and as shown for example in the embodiments of Figures 3 and 5, the absorbent core 28 can comprise a substrate layer 100, a layer of absorbent polymer material 110, a layer of adhesive 120. Typically the adhesive can be a hot melt adhesive. In an embodiment of the present invention, the layer of adhesive 120 can be typically for example a layer of fiberized hot melt adhesive 120. The substrate layer 100 can for example comprise a fibrous material.

An alternative embodiment of the present invention is shown in Figure 4. The absorbent core shown in Figure 4 can further comprise a cover layer 130. Suitable materials for the cover layer can be for example nonwoven materials.

The substrate layer 100 comprises a first surface and a second surface. Conventionally, in all the sectional views illustrated in the attached drawings the first surface of each layer is meant to correspond to the top surface, in turn, unless stated otherwise, corresponding to the wearer facing surface of the article 20 incorporating the absorbent core, while the second surface corresponds to the bottom surface, hence in turn the garment facing surface. At least portions of the first surface of the substrate layer 100 are in contact with a layer of absorbent polymer material 110. This layer of absorbent polymer material 110 comprises a first surface and a second surface, and can be typically a uniform or non uniform layer, wherein by "uniform" or "non uniform" it is meant that the absorbent polymer material 110 can he distributed over the substrate layer 100 respectively with uniform or non uniform basis weight over the area interested by the distribution. Conversely, the second surface of the layer of absorbent polymer material 110 is in at least partial contact with the first surface of the substrate layer 100. According to an embodiment of the present invention, the layer of absorbent polymer material 110 can also be a discontinuous layer that is a layer typically comprising openings, i.e. areas substantially free of absorbent polymer material, which in certain embodiments can be typically completely surrounded by areas comprising absorbent polymer material. Typically these openings have a diameter or largest span of less than 10 mm, or less than 5 mm, or 3 mm, or 2 mm, or 1.5 mm and of more than 0.5 mm, or 1 mm. At least portions of the second surface of the absorbent polymer material layer 110 are in contact with at least portions of the first surface of the substrate layer material 100. The first surface of the layer of absorbent polymer material 110 defines a certain height of the layer of absorbent polymer material above the first surface of the layer of substrate material 100. When the absorbent polymer material layer 110 is provided as a non uniform layer, typically for example as a discontinuous layer, at least some portions of the first surface of the substrate layer 100 are not covered by absorbent polymer material 110. The absorbent core 28 further comprises a layer of adhesive 120, for example typically a hot melt adhesive. This typically hot melt adhesive 120 serves to at least partially immobilize the absorbent polymer material 110. According to an embodiment of the present invention, the adhesive 120 can be typically a fiberized hot melt adhesive, i.e., being provided in fibres as a fibrous layer.

In an alternative embodiment of the present invention, as illustrated in Figure 4, the absorbent core 28 can further comprise a cover layer 130 having respective first and second surface, positioned such that the second surface of the cover layer 130 is in contact with the first surface of the layer of typically hot melt adhesive 120.

In an embodiment of the present invention comprising e.g. a non uniform layer of absorbent polymer material 110 the typically hot melt adhesive 120, for example typically provided as a fibrous layer, can be partially in contact with the absorbent polymer material 110 and partially in contact with the substrate layer 100. Figures 3 and 5 show such a structure in an exemplary embodiment of the present invention. In this structure the absorbent polymer material layer 110 is provided as a discontinuous layer, a layer of adhesive 120 is laid down onto the layer of absorbent polymer material 110, typically, for example, a layer of hot melt adhesive in fiberized form, such that the second surface of the adhesive layer 120 is in direct contact with the first surface of the layer of absorbent polymer material 110, but also in direct contact with the first surface of the substrate layer 100, where the substrate layer is not covered by the absorbent polymer material 110, i.e. typically in correspondence of the openings of the discontinuous layer of the absorbent polymer material 110. By saying "in direct contact", as well as more generally "in contact", as used herein, it is meant that there is no further intermediate component layer between e.g. the layer of adhesive 120 and the other respective layer in direct contact thereto, such as for example a further fibrous layer. It is however not excluded that a further adhesive material can be comprised between the layer of adhesive 120 and the optional cover layer 130, when present, as shown in Figure 4, or the layer of absorbent polymer material 110 or, more typically, the substrate layer 100, such as for example a supplementary adhesive material provided onto the first surface of the substrate layer 100 to further stabilize the overlying absorbent polymer material 110. "In direct contact" and "in contact" can hence be considered to comprise in this context a direct adhesive contact between the layer of hot melt adhesive 120 and another respective layer as explained above, or more in general direct and, typically, adhesive contact between two layers, e.g. the layer of absorbent polymer material and the substrate layer. This imparts an essentially three-dimensional structure to the fibrous layer of hot melt adhesive 120 which in itself is essentially a two-dimensional structure of relatively small thickness (in z-direction), as compared to the extension in x- and y-direction. In other words, the layer of adhesive 120 undulates between the first surface of the absorbent polymer material 110 and the first surface of the substrate layer 100. The areas where the layer of adhesive 120 is in direct contact with the substrate layer 100, when present according an embodiment of the present invention, are the areas of junction 140.

Thereby, in such an embodiment the adhesive 120 can provide spaces to hold the absorbent polymer material 110 typically towards the substrate layer 100, and can thereby immobilize this material. In a further aspect, the adhesive 120 can bond to the substrate 100 thus affixing the absorbent polymer material 110 to the substrate 100. Typical hot melt adhesive materials can also penetrate into both the absorbent polymer material 110 and the substrate layer 100, thus providing for further immobilization and affixation.

In the alternative embodiment representatively illustrated in Figure 4 portions of the cover layer 130 bond to portions of the substrate layer 100 via the adhesive 120. Thereby, the substrate layer 100 together with the cover layer 130 can provide spaces to immobilize the absorbent polymer material 110.

Of course, while the typically hot melt adhesive materials disclosed herein can provide a much improved wet immobilisation, i.e. immobilisation of absorbent polymer material when the article is wet or at least partially loaded, these hot melt adhesive materials can also provide a very good immobilisation of absorbent polymer material when the article is dry.

In accordance with an embodiment of the present invention, the absorbent polymer material 110 may also be optionally mixed with fibrous material, which can provide a matrix for further immobilization of the absorbent polymer material. However, typically a relatively low amount of fibrous material can be used, for example less than about 40 weight %, less than about 20 weight %, or less than about 10 weight % of the total weight of the absorbent polymer material 110, positioned within the areas of absorbent polymer material.

According to an embodiment of the present invention, in a typically discontinuous layer of absorbent polymer material 110 the areas of absorbent polymer material can be connected to one another, while the areas of junction 140 can be areas, which in an embodiment may correspond to the openings in the discontinuous layer of absorbent polymer material, as shown for example in Figure 5. The areas of absorbent polymer material are then referred to as connected areas. In an alternative embodiment, the areas of junction 140 can be connected to one another. Then, the absorbent polymer material can be deposited in a discrete pattern, or in other words the absorbent polymer material represents islands in a sea of adhesive 120. Hence, in summary, a discontinuous layer of absorbent polymer material 110 may comprise connected areas of absorbent polymer material 110, as e.g. illustrated in Figure 5, or may alternatively comprise discrete areas of absorbent polymer material 110.

The present invention, and specifically the embodiments described with reference to Figures 3, 4 and 5 can be typically used to provide the absorbent core of an absorbent article, as illustrated in Figure 1. In that case, no further materials wrapping the core, such as for example a top layer and a bottom layer are being used. With reference to the embodiments of Figure 4 the optional cover layer 130 may provide the function of a top layer and the substrate layer 100 may provide the function of a bottom layer of an absorbent core, wherein top and bottom layers respectively correspond to the body facing and garment facing surfaces of the core 28 in an absorbent article.

With reference to Figures 3, 4 and 5, according to exemplary embodiments of the present invention, the areas of direct contact between the adhesive 120 and the substrate material 100 are referred to as areas of junction 140. The shape, number and disposition of the areas of junction 140 will influence the immobilization of the absorbent polymer material 110. The areas of junction can be for example of squared, rectangular or circular shape. Areas of junction of circular shape can have a diameter of more than 0.5 mm, or more than 1 mm, and of less than 10 mm, or less than 5 mm, or less than 3 mm, or less than 2 mm, or less than 1.5 mm. If the areas of junction 140 are not of circular shape, they can be of a size as to fit inside a circle of any of the diameters given above.

The areas of junction 140, when present, can be disposed in a regular or irregular pattern. For example, the areas of junction 140 may be disposed along lines as shown in Figure 5. These lines may be aligned with the longitudinal axis of the absorbent core, or alternatively they may have a certain angle in respect to the longitudinal edges of the core. A disposition along lines parallel with the longitudinal edges of the absorbent core 28 might create channels in the longitudinal direction which can lead to a lesser wet immobilization, hence fur example the areas of junction 140 can be arranged along lines which form an angle of about 20 degrees, or about 30 degrees, or about 40 degrees, or about 45 degrees with the longitudinal edges of the absorbent core 28. Another pattern for the areas of junction 140 can be a pattern comprising polygons, for example pentagons and hexagons or a combination of pentagons and hexagons. Also typical can be irregular patterns of areas of junction 140, which also can give a good wet immobilization. Irregular patterns of areas of junction 140 can also give a better fluid handling behaviour in case of absorption of menses or blood or vaginal discharges, since fluid can start diffusing in whichever direction from any initial acquisition point with substantially the same probability of contacting the absorbent polymer material in the e.g. discontinuous layer. Conversely, regular patterns might create preferential paths the fluid could follow with lesser probability of actually contacting the absorbent polymer material.

According to the present invention the layer of adhesive 120 can comprise any suitable adhesive material. Typically, the layer of adhesive 120 can comprise any suitable hot melt adhesive material.

Without wishing to be bound by theory it has been found that those hot melt adhesive materials can be most useful for immobilizing the absorbent polymer material 110, which combine good cohesion and good adhesion behaviour. Good adhesion can typically ensure that the hot melt adhesive layer 120 maintains good contact with the absorbent polymer material 110 and in particular with the substrate material 100. Good adhesion is a challenge, namely when a nonwoven substrate material is present. Good cohesion ensures that the adhesive does not break, in particular in response to external forces, and namely in response to strain. The adhesive is subject to external forces when the absorbent product has acquired liquid, which is then stored in the absorbent polymer material 110 which in response swells. An exemplary adhesive should allow for such swelling, without breaking and without imparting too many compressive forces, which would restrain the absorbent polymer material 110 from swelling. It may be desirable that the adhesive does not break, which would deteriorate the wet immobilization. Exemplary suitable hot melt adhesive materials can be as described in the already mentioned patent application EP 1447067, particularly at sections [0050] to [0063].

The adhesive material, typically a hotmelt adhesive material, can be typically present in the form of fibres throughout the core, being provided with known means, i.e. the typically hot melt adhesive can be fiberized. Typically, the fibres can have an average thickness from about 1 µm to about 100 µm, or from about 25 µm to about 75 µm, and an average length from about 5 mm to about 50 cm. In particular the layer of typically hot melt adhesive material can be provided such as to comprise a net-like structure.

To improve the adhesiveness of the typically hot melt adhesive material 120 to the substrate layer 100 or to any other layer, in particular any other non-woven layer, such layers may be pre-treated with an auxiliary adhesive.

In particular, typical parameters of a hot melt adhesive in accordance with the present invention can be as follows.

In an aspect, the loss angle tan Delta of the adhesive at 60°C should be below the value of 1, or below the value of 0.5. The loss angle tan Delta at 60°C is correlated with the liquid character of an adhesive at elevated ambient temperatures. The lower tan Delta, the more an adhesive behaves like a solid rather than a liquid, i.e. the lower its tendency to flow or to migrate and the lower the tendency of an adhesive superstructure as described herein to deteriorate or even to collapse over time. This value is hence particularly important if the absorbent article is used in a hot climate.

In a further aspect, typical hot melt adhesives in accordance with the present invention may have a sufficient cohesive strength parameter γ. The cohesive strength parameter γ is measured using the Rheological Creep Test as referred to hereinafter. A sufficiently low cohesive strength parameter γ is representative of elastic adhesive which, for example, can be stretched without tearing. If a stress of τ = 1000 Pa is applied, the cohesive strength parameter γ can be less than 100%, less than 90%, or less than 75%. For a stress of τ = 125000 Pa, the cohesive strength parameter γ can be less than 1200%, less than 1000%, or less than 800%.

In a further aspect of the present invention, it has been found that absorbent cores can be formed by combining two core structures e.g. as shown in Figure 3 and as described in the context thereof. The absorbent core hence can comprise two substrate layers 100, two layers of absorbent polymer material 110 and two layers of adhesive material 120. When two discontinuous layers of an absorbent polymer material 110 arc used, they could be arranged in such a way that the absorbent polymer material of the one layer faces the areas of junction 140 of the other layer. In an alternative embodiment, however, the areas of junction 140 can be offset and do not face each other. Typically, when two core structures are joined, this can be typically done such that the first surface of the substrate layer 100 of the first core structure faces the first surface of the substrate layer 100 of the second core structure.

Typically the absorbent polymer material 110 for the absorbent cores according to the present invention can comprise absorbent polymer particles, known in the art e.g. as superabsorbent materials, or as absorbent gelling materials, or also as hydrogel forming materials, as referred to in the Background of the Invention. Typically absorbent polymer particles can have a selected average particle size,

According to the present invention, the layer of absorbent polymer material 110 comprises porous absorbent polymer particles 110'. Typically the layer of absorbent polymer material 110 comprises at least about 40% by weight, or about 60% by weight, or also about 80% by weight porous absorbent polymer particles 110'. In an embodiment of the present invention, the layer of absorbent polymer material 110 can be entirely constituted of porous absorbent polymer particles 110'.

Porous absorbent polymer materials, typically porous absorbent polymer particles comprised in the absorbent core of the present invention, can typically comprise substantially water-insoluhle, water-swellable polymer particles, also known as superabsorbent polymer particles, which are also porous. As used herein, and as commonly known in the art, the term "porous" means a structure forming walls surrounding and defming cellular voids in absorbent polymer materials, e.g. absorbent polymer particles, when substantially dry. Cellular voids can typically define an open cell structure, but alternatively also a closed cell structure. In general a porous structure of an absorbent polymer material can provide the porous absorbent polymer material with low density and, generally, with a higher specific surface area. Typically, under microscopic observations, walls formed in a porous absorbent polymer material, typically in absorbent polymer particles, can show e.g. a sponge-like appearance, or a withered leaf-like appearance, possibly depending on how the porous absorbent polymer material, typically in particle form, has been manufactured.

Porous absorbent polymer materials, typically porous absorbent polymer particles, can be obtained according to various manufacturing methods. Methods involving use of a blowing agent during polymerization or crosslinking, are described for example in US 4,529,739, US 4,649,164, JP 62-34042, JP 2-60681, JP 2-54362, US 5,118,719, US 5,154,713, US 5,314,420, US 6,251,960, US 7,163,966, US 4,808,637, JP 59-18712, US 4,552,938, US 4,654,393, US 4,703,067, US 5,328,935, US 5,338,766. Methods involving addition of a blowing agent after polymerization are described for example in JP 56-13906, JP 57-182331, JP 57-208236. Further methods for providing porous absorbent polymer particulate materials are also described in WO 91/02552, WO 93/24153, US 5,002,986, US 5,300,565, US 5,140,076, US 4,732,968, US 4,742,086, US 5,354,290, US 5,403,870, US 5,985,432.

As alternative, porous absorbent polymer particles may be also obtained by agglomeration of smaller particles, which in turn can possibly, but not necessarily, be porous, wherein pores can typically comprise the voids among the agglomerated particles.

According to an embodiment of the present invention, the porous absorbent polymer particles can have a bulk density of from about 0.01 g/cm³ to about 0.4 g/cm³, or from about 0.03 g/cm³ to about 0.35 g/cm³, or also from about 0.06 g/cm³ to about 0.3 g/cm³. The bulk density of a material in powder or particulate form, also known as apparent density, refers to the weight per unit volume of the material, including voids inherent in the material. It can be measured according to standard methods known in the art; for example, in the context of the present invention, the bulk/apparent density can be measured according to the method referred to hereinafter, in the Test Methods section.

According to an embodiment of the present invention, porous absorbent polymer materials, typically in particle form, can be selected among polyacrylates and polyacrylate based materials, such as for example partially neutralized, crosslinked polyacrylates.

In an embodiment of the present invention the absorbent polymer material 110, in turn comprising the porous absorbent particles 110', in the absorbent core 28 is present throughout the area of the absorbent core in an average basis weight of less than about 250 g/m², or of less than about 220 g/m², or from about 60 g/m² to about 180 g/m², or from about 100 g/m² to about 160 g/m². Average basis weights for the absorbent polymer material 110 of up to about 300 g/m², or up to about 400 g/m², or also up to about 500 g/m² can also be used according to the present invention. An average basis weight is typically based on the whole area of the zone of application, i.e. interested hy the layer of absorbent polymer material, and hence comprising possible openings included in an e.g. discontinuous layer. Typically, the absorbent polymer material 110 can constitute at least about 45%, or at least about 50%, or at least about 55%, by weight of the absorbent core, wherein the absorbent core can typically correspond to the embodiments described with reference to Figures 3, 4, and 5, hence comprising the substrate layer, the layer of absorbent polymer material, the layer of thermoplastic material, the layer of foam material, the optional cover layer if present, and any other material possibly comprised within this structure, such as for example the additional fibrous material mentioned above or the additional adhesive material.

In an embodiment of the present invention wherein the layer of absorbent polymer material 110 can be entirely comprised of porous absorbent polymer particles 110', the above basis weights indicated for the layer of absorbent polymer material 110 can correspond to the actual basis weights of the porous absorbent polymer particles 110', the further general considerations remaining the same.

The absorbent polymer particles, typically both porous and, if present, also non porous absorbent polymer particles of the layer of absorbent polymer material 110, can typically have a selected average particle size from about 200 µ to about 600 µ, or from about 300 µ to about 500 µ.

The average particle size of a material in particulate form, namely for example the absorbent polymer material and the porous absorbent polymer particles, can be determined as it is known in the art, for example by means of dry sieve analysis. Optical methods, e.g. based on light scattering and image analysis techniques, can also be used. A method is described hereinafter in the Test Methods section.

It is believed that the layer of adhesive 120, typically a hot melt adhesive, provided onto the layer of absorbent polymer material 110, and in direct contact therewith, can provide an effective absorbent structure, stabilizing and containing the absorbent polymer material onto the substrate layer 100, both in dry, and also in wet conditions. This can be particularly relevant when the layer of absorbent polymer material 110 is provided by absorbent polymer particles, wherein the occurrence of loose absorbent polymer particles within the absorbent core structure is minimized. However, the layer of adhesive 120, typically a hot melt adhesive, for example typically provided in fibrous form, indeed covers at least part of the outer surface of the absorbent polymer material, for example absorbent polymer particles, and hence can create some interposition between the absorbent polymer material, e.g. the absorbent polymer particles, and the incoming fluid meant to be absorbed by the absorbent polymer material itself. This in turn may have some effect on the fluid management capability of the absorbent polymer material, and in turn of the absorbent core, for example in terms of a slightly reduced acquisition capacity. It is believed that a porous absorbent polymer material, particularly porous absorbent polymer particles, is less subject to this influence of the adhesive material. Without being bound to any theory, possibly the layer of adhesive material, for example in fibrous form, covers only partially the solid external surface of the porous absorbent polymer particles, while pores themselves can compensate for this sort of shielding effect by providing a greater surface for fluid acquisition and absorption, which moreover is less subject to be hindered by the adhesive material as it is located internally to the particles themselves, and hence less subject to a direct contact with the adhesive material. The effect is even more pronounced when the adhesive material is for example provided in fibrous form, as the fibres of adhesive material are believed to at least partially span across the pores themselves, possibly leaving the remaining of the opening of the pore pervious for fluid acquisition.

Exemplary materials for the substrate layer 100 according to an embodiment of the present invention can comprise nonwoven materials comprising synthetic fibres, or natural fibres, or mixtures thereof, such as for example carded nonwovens, or more typically airlaid or wetlaid fibrous materials, such as for example latex or thermal bonded airlaid fibrous materials, comprising synthetic and natural fibres, such as for example cellulose fibres.

According to an embodiment of the present invention, the substrate layer 100 can comprise a fibrous material comprising cellulose or cellulose derivative fibres, typically for example from about 40% to about 100% by weight of cellulose or cellulose derivative fibres, or from about 50% to about 95% by weight of cellulose or cellulose derivative fibres, or also from about 60% to about 90% by weight of cellulose or cellulose derivative fibres. In a core structure according to the present invention a substrate layer 100 constituted by a fibrous material comprising a substantial percentage of cellulose fibres can provide an advantage in terms of liquid distribution towards the liquid fraction which is not immediately absorbed by the upper layer of absorbent polymer material 110, and is directly acquired by the substrate layer 100. In general cellulose or cellulose derivative fibres can create some competition in fluid absorption with more traditional absorbent polymer materials, which can translate in a slower and somehow less efficient fluid handling, since the fluid may be acquired and absorbed more rapidly by a substrate layer 100 containing a substantial amount of cellulose fibres, compared to the absorbent polymer material itself, hence using less than optimally its theoretical absorbent capacity. It is believed that this competition effect can be substantially reduced when the layer of absorbent polymer material in an absorbent core structure according to an embodiment of the present invention comprises porous absorbent polymer particles, therefore taking better advantage of the characteristics of the two respective materials, with a synergistic effect.

Basis weights for the materials of the substrate layer 100 can typically range from about 10 g/m² to about 120 g/m², or from about 40 g/m² to about 100 g/m², or also from about 50 g/m² to about 80 g/m².

Exemplary materials for the optional cover layer 130 can be provided by nonwoven materials comprising synthetic fibres, such as polyethylene (PE), polyethylene terephthalate (PET), polypropylene (PP). As the polymers used for nonwoven production are inherently hydrophobic, they can be typically coated with hydrophilic coatings, for example with durably hydrophilic coatings to provide permanently hydrophilic nonwovcns. Other nonwoven materials for the optional cover layer 130 can comprise composite structures such as a so called SMS material, comprising a spunbonded, a melt-blown and a further spunbonded layer. Basis weights for the materials of the cover layer 130 can typically range from 5 g/m² to 80 g/m², or from 10 g/m² to 60 g/m², or also from 20 g/m² to 40 g/m².

According to the present invention, the absorbent core can provide a more efficient fluid management, in terms of acquisition, immobilization and absorption and a better comfort, during the entire wearing time of the article, as explained above, which can be particularly useful in case of complex body fluids such as menses or blood. Overall, this increased efficiency in the composite structure according to the present invention can translate in a more effective exploitation of the absorbent capacity of the absorbent polymer material, also in presence of problematic body fluids such as menses or blood or vaginal discharges, and possibly also in a more efficient use of the entire structure of the absorbent core.

This is achieved in a structure which is typically thin and flexible, yet capable of employing more completely the absorption and immobilization capacity of the different materials, and having improved fit and resilience during absorption and therefore incrcascd comfort during use.

According to an embodiment of the present invention the absorbent polymer material, and more particularly the porous absorbent polymer particles, can be selected among the polyacrylate based polymers described in the PCT Patent Application WO 07/047598, which are polyacrylate based materials very slightly crosslinked, or substantially not crosslinked at all, this further improving the above mentioned synergistic effect. Particularly, said polyacrylate based materials can have an extractable fraction of at least about 30% by weight, between about 30% and about 80% by weight, or between about 32% and about 70% by weight, evaluated according to the Extractables test method described in the above referenced application. Alternatively, said polyacrylate based materials can have a retention capacity of at least about 30 g/g, at least about 35 g/g, or at least about 40 g/g, evaluated according to the Centrifuge Retention Capacity test described in the above referenced application. The absorbent polymer material can also be selected among the polyacrylate based polymers described in the PCT Patent Application WO 07/046052. Said polymers in fact are particularly effective in absorbing complex body fluids such as menses or blood, and upon absorption of such fluids do not generally show a marked swelling, followed by gel blocking, like traditional superabsorbcnts, but rather act to a certain extent as thickeners of the body fluid, immobilizing it as a sort of gelatinous mass within the absorbent structure, for example in the interstices among the fibres, without causing substantial swelling and in turn a sensible increase of the overall thickness of the absorbent core. Said polymers can be provided in form of porous absorbent polymer particles according to known methods.

According to the present invention, the absorbent core 28 can fully constitute the absorbent element in an absorbent article, or can be complemented with additional layers. Also, an absorbent article comprising an absorbent core according to the present invention can further comprise a fibrous acquisition layer between the absorbent core 28 and the topsheet. According to an embodiment of the present invention the acquisition layer can for example comprise fibrous nonwoven materials made by air laying or wet laying of synthetic fibres such as polyethylene (PE), polyethylene terephthalate (PET), or polypropylene (PP), similarly to the cover layer 130 of the absorbent core 28 of the present invention.

Exemplary materials for the fluid acquisition layer could comprise spunbonded or carded nonwoven materials, or airlaid materials such as for example latex bonded or thermal bonded airlaid materials. Basis weights can typically range from about 10 g/m² to about 60 g/m², or from about 25 g/m² to about 40 g/m².

According to another alternative embodiment of the present invention the absorbent article can comprise a further fibrous layer comprised between the absorbent core 28 and the backsheet, i.e. typically provided at the garment facing surface of the core. This optional layer can be provided by similar fibrous materials as those already described for the substrate layer 100 of the absorbent core of the present invention. This optional fibrous layer according to this further embodiment of the present invention can act as an added wicking layer receiving and distributing excess fluid which might not be fully retained by the absorbent core 28. The presence of cellulose fibres can make the layer particularly effective in acquiring and diffusing the fraction of body fluids like menses or blood which is not completely absorbed by the absorbent polymer material of the absorbent core 28.

Further materials, also typically in particle form, can be comprised in the layer of absorbent polymer material, for example known odour control materials, or inert materials such as silica.

The absorbent polymer material for the absorbent cores according to the present invention, typically comprising absorbent polymer particles, according to a further embodiment of the present invention, can have a permeability, as expressed by the saline flow conductivity of the absorbent polymer material, greater than 10, 20, 30 or 40 SFC- units, where 1 SFC unit is 1 x 10⁻⁷ (cm³ x s) / g. Saline flow conductivity is a parameter well recognised in the art and is to be measured in accordance with the test disclosed in EP 752 892 B.

### Backsheet

The absorbent article of Figure 1 comprising the absorbent core according to the present invention can also comprise a backsheet 40. The backsheet may be used to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to an embodiment of the present invention can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

### Test Methods

### Average particle size

The method is similar to that described in patent US 6,096,299 at lines 1-35 of column 3 can be used, slightly modified as explained below.

The average particle size of a material can be determined by a mechanical sieve shaker method using an electromagnetic sieve shaker Fritsch ® Analysette 3, or equivalent, with six Tyler Screen Standard analytical sieves (20, 32, 42, 65, 100 and 150 Mesh, respectively corresponding to 841, 500, 354, 210, 149 and 105 µm) and one sieve pan. The selected sieves are nested with the coarsest sieve at the top and the solid pan at the bottom, then the test sample (100 g of material) is placed on the top sieve and the nest is closed with a cover. The shaker is operated for 15 minutes continuously with an amplitude of vibrations of 1 mm and finally, after the completion of the agitation, the material retained on each sieve is weighed separately. The material which has passed through the finest sieve into the pan is also weighed.

These weights and the weight of the original test sample are used to calculate the average particle size of the test sample. In order to get the average particle size the percentage retained on each sieve is calculated by dividing the "total weight coarser" than that sieve by the total weight of the test sample. The total weight coarser includes the material retained on that particular sieve plus all material on all coarser sieves. This cumulative percentage represents the total percentage of the test sample coarser than the aperture of that particular sieve.

The data is plotted on a sieve analysis graph where the abscissa represents the sieve sizes (on a logarithmic scale) and the ordinate the percentages retained (on a linear scale). By interpolation on the sieve analysis graph the sieve size corresponding to a percentage of 50% retained can be evaluated, and this size is taken as the average particle size of the sample.

### Apparent density

The apparent density, also known as bulk density, of the absorbent polymer material, typically in particle form, can be measured for example according to the following method. After the absorbent material has been totally dried as said below, the material is packed in a 10 cm³ graduated cylinder. During the packing, the graduated cylinder is kept without being tapped. The weight of the packed sample is measured and the bulk density is obtained by dividing the measured sample weight by the sample volume (10 cm³).

It has to be noted that both the test for the average particle size and the test for the apparent/bulk density shall he conducted in a controlled environment, typically at 23±2°C and 50±10% RH. The sample material for both tests shall be dried in an oven for three hours at 105°C, then kept in a closed container and allowed to equilibrate to the ambient laboratory temperature. Care shall be taken to test the sample material quickly, i.e. typically in no more than 45 minutes after removal from the closed container in order to minimize moisture gain in the controlled laboratory environment.

### Rheological Creep Test

The Rheological Creep Test mentioned hereinabove for measuring the cohesive strength parameter γ is as described in the copending patent application EP 1447067, assigned to the Procter & Gamble Company.

### Alternative sample preparation for all tests herein when starting from an absorbent article_{.}

When starting from an article comprising the absorbent polymer material, in turn comprising the porous absorbent polymer particles, said material can be isolated with known means, typically from the layer of thermoplastic material and the substrate layer, in order to be tested. Typically, in a disposable absorbent article the topsheet can be removed from the backsheet and the absorbent core can be separated from any additional layers, comprising the optional cover layer, if present. The absorbent polymer material can be removed from the substrate layer and the layer of thermoplastic material, e.g. mechanically if possible, or by use of a suitable solvent, in case e.g. the thermoplastic material is a hot melt adhesive. Particles of absorbent polymer material can be hence isolated from other elements of the core e.g. by washing with a suitable solvent which does not interact with the absorbent polymer material, as can be readily determined by the man skilled in the art. The solvent is then let to evaporate and the porous absorbent polymer particles can be separated from the non porous absorbent polymer material, if present, with known means, and collected, for example from a plurality of articles of the same type, in the necessary amounts to run the tests.

Alternatively, the average particle size of the absorbent polymer material can be measured for example with optical means, e.g. based on light scattering and image analysis techniques, directly on the structure typically comprising the substrate layer, the absorbent polymer material, and the layer of thermoplastic material.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent core for an absorbent article, said core comprising a substrate layer, said substrate layer comprising a first surface and a second surface,
said absorbent core further comprising a layer of absorbent polymer material,
said layer of absorbent polymer material comprising a first surface and a second surface,
said absorbent core further comprising a layer of adhesive,
said layer of adhesive comprising a first surface and a second surface,
wherein said layer of absorbent polymer material is comprised between said layer of adhesive and said substrate layer;
said second surface of said layer of absorbent polymer material is in contact with said first surface of said substrate layer;
and said first surface of said layer of absorbent polymer material is in contact with said second surface of said layer of adhesive,
**characterized in that**
said layer of absorbent polymer material comprises porous absorbent polymer particles.

2. An absorbent core according to claim 1, wherein at least 40% by weight, preferably at least 60% by weight, more preferably at least 80% hy weight, of said absorbent polymer material are porous absorbent polymer particles.

3. An absorbent core according to claim 1, wherein said absorbent polymer material is entirely constituted of porous absorbent polymer particles.

4. An absorbent core according to any preceding claim, wherein said porous absorbent polymer particles have an average particle size from 200 µ to 600 µ, preferably from 300 µ to 500 µ.

5. An absorbent core according to any preceding claim, wherein said substrate layer is a fibrous material comprising from 40% to 100% by weight, preferably from 50% to 95% by weight, more preferably from 60% to 90% by weight, of cellulose or cellulose derivative fibres.

6. An absorbent core according to any preceding claim, wherein said porous absorbent polymer particles are present in an average basis weight of less than 250 g/m², or of less than 220 g/m², or from 60 g/m² to 180 g/m², or from 100 g/m² to 160 g/m².

7. An absorbent core according to any preceding claim, wherein said porous absorbent polymer particles have a bulk density of from 0.01 g/cm³ to 0.4 g/cm³, or from 0.03 g/cm³ to 0.35 g/cm³, or also from 0.06 g/cm³ to 0.3 g/cm³.

8. An absorbent core according to any preceding claim, wherein said layer of absorbent polymer material is non uniform, and whcrcin said second surface of said non uniform layer of absorbent polymer material is in at least partial contact with said first surface of said substrate layer, and portions of said second surface of said layer of thermoplastic material are in contact with said first surface of said substrate layer and portions of said second surface of said layer of thermoplastic material are in contact with said first surface of said non uniform layer of absorbent polymer material.

9. An absorbent article comprising a liquid permeable topsheet, a backsheet and an absorbent core according to any preceding claim comprised therebetween.
